# EUROPEAN PATENT SPECIFICATION

(11) **EP 0 613 670 B1**
(45) Date of publication and mention of the grant of the patent: **31.05.2000**
(21) Application number: 94301407.6
(22) Date of filing: 28.02.1994
(51) Int. Cl.: A61F 13/02

(54) **Process for making an adhesive dressing**
Verfahren zur Herstellung eines Klebeverbandes
Procédé de fabrication d'un pansement adhésif

(30) Priority: 01.03.1993 ZA 931440
(43) Date of publication of application: 07.09.1994
(73) Proprietor: JOHNSON & JOHNSON CONSUMER PRODUCTS, INC., Skillman, New Jersey 08558-9418 (US)
(72) Inventor: Sperinck, Peter George, Randburg, Transvaal Province (ZA); Charlton, Douglas John, Randburg, Transvaal Province (ZA)
(74) Representative: Fisher, Adrian John

(56) References cited:
- CH-A- 676 197
- US-A- 2 633 128
- US-A- 2 862 846

## Description

This invention relates to a process for making an adhesive dressing.

CH-A-676197 discloses a process for making an adhesive bandage, in which adhesive is continuously applied to a backing strip and a release paper is then applied to cover the adhesive. In a subsequent operation, spots of adhesive are applied to a different region of the backing strip, and absorbent pads are then adhered to such spots of adhesive.

US-A-2862846 discloses the manufacture of adhesive bandages from a laminate comprising a carrier paper, a plastic film, an adhesive layer on the plastic film, and a silicone coated release paper.

According to the invention, there is provided a process for making an adhesive dressing, which comprises
passing a backing strip continuously through an adhesive application zone;
applying an adhesive to one side of the backing strip as it passes continuously through this zone, to form an adhesive coated strip;
passing the adhesive coated strip from the adhesive application zone continuously through an adhesive conditioning zone, to condition the adhesive;
before or after the adhesive conditioning zone, passing the adhesive coated strip continuously through a pad application zone where at least one pad is applied to the adhesive coated side of the backing strip in such fashion that portions of the adhesive are not covered by the pad, thereby to form a padded strip; and
feeding the padded strip continuously and directly to a release paper application zone, where release paper is applied over at least the adhesive, to form a continuous adhesive dressing.

The process of the invention is thus characterized thereby that it is continuous, ie the strip passes continuously and directly from one zone to the next, without intermediate taking up thereof into a roll.

The adhesive may be a pressure sensitive adhesive.

In one embodiment of the invention, the adhesive may be a curable adhesive such as a solvent or emulsion based adhesive. Curing of the adhesive may then take place in the adhesive conditioning zone.

The pad may then be of curable foam material, as hereinbefore described, with the adhesive coated strip passing through the pad application zone before passing through the conditioning or curing zone, so that curing of the foam material is also effected in the conditioning or curing zone. In one version of this embodiment, the foam material may be applied continuously to the backing strip, eg so that a single pad extending along the strip is thereby provided. However, in another version, the foam material may be applied intermittently to the backing strip, eg so that a plurality of pads spaced longitudinally and/or laterally on the strip are thereby provided.

Instead, the pad(s) may be of fabric. In one version of the invention, the fabric may be applied as a continuous strip to the adhesive coated strip. However, in another version of the invention, individual fabric pads may be applied intermittently to the adhesive coated strip. The fabric, when used as the pad material, is preferably, but not necessarily always, absorbent.

The pad(s) can instead be of any other suitable material such as fibrous material or a gel.

The adhesive coated strip may pass through the curing zone before application of the pad(s) thereto.

In another embodiment of the invention, the adhesive may be a hot-melt adhesive applied to the backing strip at elevated temperature. Cooling of the adhesive may then be effected in the conditioning zone. The pad(s) may then, irrespective of whether they are in the form of a curable foam material, or a non-curable fabric, as hereinbefore described, preferably be applied to the adhesive coated strip after the adhesive has been cooled. Howver, when the pad(s) is/are of curable foam material, the process may include passing the padded strip through a curing stage prior to entering the release paper application zone.

The process may include passing the continuous adhesive dressing from the release paper application zone to a cutting zone, and cutting it into individual dressings of a desired shape and size in the cutting zone. The cutting may be effected by means of a die cutting arrangement, eg by cutting a plurality of dressings side-by-side from the strip.

The backing strip or layer may be cellulose based, may be in the form of a polymeric film, may be in the form of either a knitted or a non-woven fabric, may be of stretch or non-stretch form, or may be combinations thereof, as desired.

When a foam material is used for the pad(s), it is preferably a SBR styrene butadiene rubber or a polyurethane foam. However, any other foam which can be coated as a relatively thin layer can be used. The foam may contain absorbency enhancing materials, such as acrylic superabsorbent powder. It may also contain a medication such as silver sulphur diazine and/or a facing layer such as a plastic net or a non-woven fabric.

The pad can thus be continuous, ie extending along the strip with the adhesive coated zone(s) located alongside it. Instead, individual pads, spaced from one another along and across the strip, can be provided.

The invention will now be described by way of example with reference to the accompanying diagrammatic drawings.

In the drawings,
FIGURE 1 shows a simplified flow diagram of a process for making an adhesive dressing, according to one embodiment of the invention;
FIGURE 2 shows a side view of an adhesive dressing strip formed in the process of Figure 1;
FIGURE 3 shows a side view of another adhesive dressing strip which can be formed in the process of Figure 1;
FIGURE 4 shows a plan view of a cutting die for effecting the cutting operation in Figure 1;
FIGURE 5 shows a plan view of an adhesive dressing formed by the process of Figure 1; and
FIGURES 6, 7 and 8 shows simplified diagrams similar to Figure 1, of processes for making an adhesive dressing according to other embodiments of the invention.

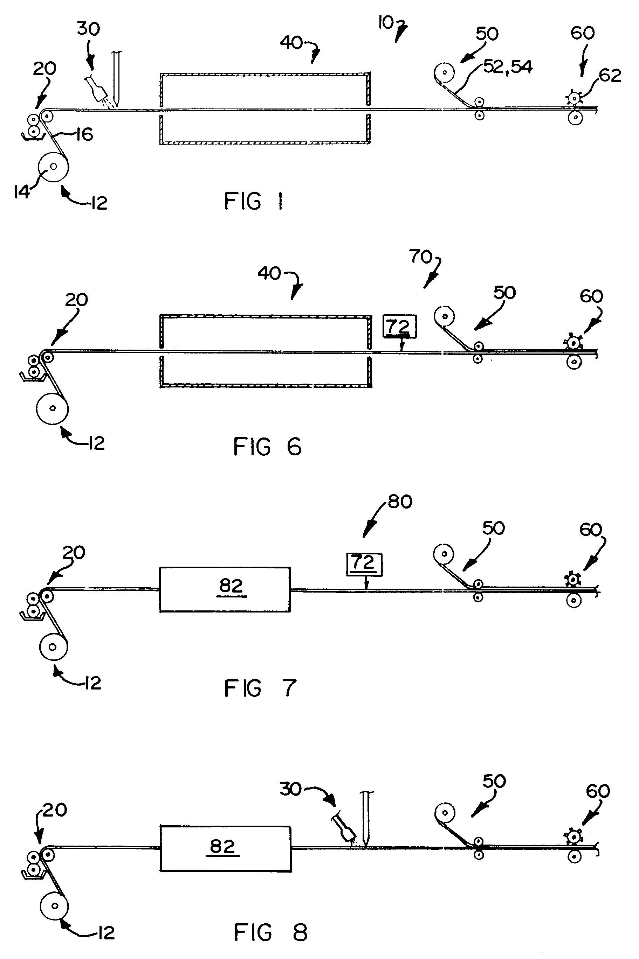

Referring to Figures 1 to 5, reference numeral 10 generally indicates a process for making an adhesive dressing, according to a first embodiment of the invention.

The process 10 includes a backing strip feed zone 12 which contains a roll 14 of backing strip 16. The backing strip 16 can be cellulose based, can be in the form of a polymeric strip, can be knitted or non-woven, can be stretch or non-stretch, or can be combinations thereof. In the zone 12 is also provided feed means (not shown) for continuously feeding the backing strip 16 from the roll 14 to an adhesive application zone, generally indicated by reference numeral 20.

In the zone 20 a curable adhesive, such as a solvent or emulsion based adhesive, is applied as a layer 22 to one side of the backing strip 16, as the backing strip 16 passes continuously through the zone 20.

From the zone 20 the adhesive coated backing layer passes to a foam coating zone 30 where a foam material, such as polyurethane foam containing absorbency enhancing material such as acrylic superabsorbent powder is applied as a pad 32 to the adhesive coated side of the backing strip or layer 16. The pad 32 can be applied intermittently, or continuously, along the strip 16, depending on the eventual dressing required.

From the zone 30 the strip passes to a curing/drying zone 40 where both the adhesive and the foam are cured/dried at elevated temperature.

The strip 16 then passes to a release paper application or laminating zone 50, where overlapping release paper layers 52, 54 are applied over the pad 32 and the adhesive layer or coating 22, while the strip 16 passes continuously therethrough.

Finally, the continuous adhesive dressing strip thus formed passes to a cutting zone 60 where it is cut, by means of a rotating cylindrical die 62, having raised cutting edges 64 defining desired dressing shapes and sizes on its outer surface, for cutting the strip into individual dressings of said desired shape and size.

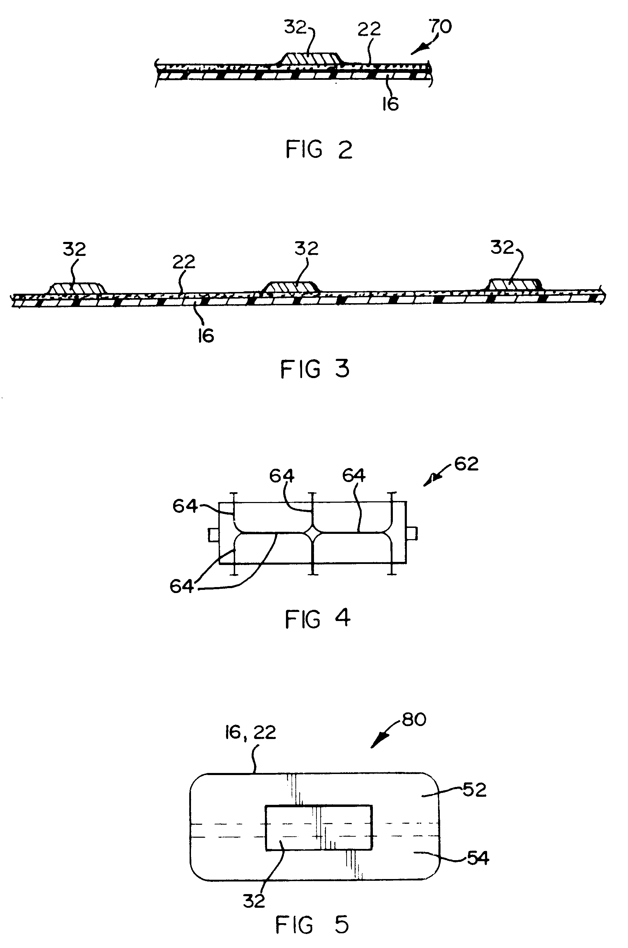

Thus, by means of the process 10, dressing strips 70, as indicated in Figure 2, can be formed. The dressing strips 70 thus comprise the backing strip 16, the adhesive layer 22 and the foam pad 32. Instead, a number of dressings can be formed side-by-side across the width of the strip 16, as indicated in Figure 3.

After the adhesive dressing strip has been cut in the cutting zone, final dressings 80 as indicated in Figure 5, are produced.

Referring to Figures 6, 7 and 8, parts of the processes depicted therein which are the same or similar to those of Figure 1, are indicated with the same reference numerals.

In Figure 6, reference numeral 70 generally indicates a process according to a second embodiment of the invention for making an adhesive dressing.

In the process 70, the foam coating zone 30 is dispensed with. Instead, individual absorbent fibrous material pads 32 are applied intermittently in a pad application zone 72 located after the curing/drying zone 40.

In Figure 7, reference numeral 80 generally indicates a process according to a third embodiment of the invention for making an adhesive dressing.

In the process 80, a hot melt adhesive is applied, in the zone 20, to the backing strip 16 at elevated temperature. The zones 30, 40 are then dispensed with. Instead, the adhesive coated strip passes through a cooling zone or stage 82 where the adhesive is cooled, whereafter individual absorbent fibrous material pads are applied thereto in the zone 72.

In Figure 8, reference numeral 90 generally indicates a process according to a fourth embodiment of the invention for making an adhesive dressing.

In the process 90, a hot melt adhesive is also used, with the drying/curing stage 40 of Figure 1 being replaced by the cooling stage 82. However, instead of having the pad application zone 72 of the process 80 of Figure 6, the foam coating zone 30 of Figure 1, located after the cooling stage 82 and ahead of the release paper application zone 50, is provided. If desired, curing/drying stage (not shown), similar to the stage 40, can be provided after the zone 30 and ahead of the zone 50.

The Applicant believes that with the processes 10, 70, 80 and 90, adhesive dressings can be made rapidly and effectively.

## Claims

1. A process for making an adhesive dressing, comprising
passing a backing strip (16) continuously through an adhesive application zone (20);
applying an adhesive to one side of the backing strip (16) as it passes continuously through this zone (20), to form an adhesive coated strip;
characterized in that it comprises
passing the adhesive coated strip from the adhesive application zone (20) continuously through an adhesive conditioning zone (40), to condition the adhesive;
before or after the adhesive conditioning zone (40), passing the adhesive coated strip continuously through a pad application zone (30) where at least one pad (32) is applied to the adhesive coated side of the backing strip in such fashion that portions of the adhesive are not covered by the pad, thereby to form a padded strip; and
feeding the padded strip continuously and directly to a release paper application zone (50), where release paper (52, 54) is applied over at least the adhesive, to form a continuous adhesive dressing.

2. A process according to Claim 1, characterized in that the adhesive is a pressure sensitive curable adhesive, with curing of the adhesive then taking place in the adhesive conditioning zone (40).

3. A process according to Claim 2, characterized in that the pad (32) is of curable foam material, with the adhesive coated strip passing through the pad application zone (30) before passing through the conditioning zone (40), so that curing of the foam material is also effected in the conditioning zone (40).

4. A process according to Claim 3, characterized in that the foam material is applied continuously to the backing strip (16).

5. A process according to Claim 3, characterized in that the foam material is applied intermittently to the backing strip (16).

6. A process according to Claim 2, characterized in that the pad (32) is of absorbent fabric and is applied as a continuous strip to the adhesive coated strip (16).

7. A process according to Claim 2, characterized in that individual absorbent fabric pads (32) are applied intermittently to the adhesive coated strip (16).

8. A process according to Claim 6 or Claim 7, characterized in that the adhesive coated strip passes through the curing zone (40) before application of the pad(s) (32) thereto.

9. A process according to Claim 1, characterized in that the adhesive is a hot-melt adhesive applied to the backing strip (16) at elevated temperature, with cooling of the adhesive being effected in the conditioning zone (40) and with the application of the pad (32) to the adhesive coated strip being after the adhesive has been cooled.

10. A process according to Claim 9, characterized in that the pad (32) is of curable foam material, with the process including passing the padded strip through a curing stage (40) prior to entering the release paper application zone (50).

11. A process according to any one of Claims 1 to 10 inclusive, characterized in that it includes passing the continuous adhesive dressing from the release paper application zone (50) to a cutting zone (60), and cutting it into individual dressings of a desired shape and size in the cutting zone.

12. A process according to Claim 11, characterized in that the cutting is effected by means of a die cutting arrangement (62).

## Patentansprüche

1. Verfahren zum Herstellen eines Klebeverbandes, welches umfaßt:
kontinuierliches Leiten eines Verstärkungsstreifens (16) durch eine Klebstoffauftragezone (20);
Auftragen eines Klebstoffes auf eine Seite des Verstärkungsstreifens (16), während er diese Zone (20) kontinuierlich durchläuft, um einen mit Klebstoff beschichteten Streifen zu bilden;
dadurch gekennzeichnet, daß es umfaßt:
kontinuierliches Leiten des mit Klebstoff beschichteten Streifens von der Klebstoffauftragezone (20) durch eine Klebstoffkonditionierzone (40), um den Klebstoff zu konditionieren;
kontinuierliches Leiten des mit Klebstoff beschichteten Streifens vor oder nach der Klebstoffkonditionierzone durch eine Auflagen-Aufbringzone (30), in der mindestens eine Auflage (32) auf die mit Klebstoff beschichtete Seite des Verstärkungsstreifens derart aufgebracht wird, daß Abschnitte des Klebstoffes nicht durch die Auflage bedeckt werden, wodurch ein mit einer oder mehreren Auflagen versehener Streifen gebildet wird; und
kontinuierliches und direktes Leiten des mit Auflagen versehenen Streifens zu einer Schutzpapier-Aufbringzone (50), in der Schutzpapier (52, 54) zumindest über dem Klebstoff aufgebracht wird, um einen Endlos-Klebeverband zu erzeugen.

2. Verfahren nach Anspruch 1, dadurch gekennzeichnet, daß der Klebstoff ein selbstklebender aushärtbarer Klebstoff ist, wobei das Aushärten des Klebstoffes dann in der Klebstoffkonditionierzone (40) stattfindet.

3. Verfahren nach Anspruch 2, dadurch gekennzeichnet, daß die Auflage (32) aus einem aushärtbaren Schaummaterial besteht, wobei der mit Klebstoff beschichtete Streifen die Auflagen-Aufbringzone (30) durchläuft, bevor er die Konditionierzone (40) durchläuft, so daß auch das Aushärten des Schaummaterials in der Konditionierzone (40) erfolgt.

4. Verfahren nach Anspruch 3, dadurch gekennzeichnet, daß das Schaummaterial kontinuierlich auf den Verstärkungsstreifen (16) aufgebracht wird.

5. Verfahren nach Anspruch 3, dadurch gekennzeichnet, daß das Schaummaterial mit Unterbrechungen auf den Verstärkungsstreifen (16) aufgebracht wird.

6. Verfahren nach Anspruch 2, dadurch gekennzeichnet, daß die Auflage (32) aus absorbierendem Stoff besteht und als ein kontinuierlicher Streifen auf den mit Klebstoff beschichteten Streifen (16) aufgebracht wird.

7. Verfahren nach Anspruch 2, dadurch gekennzeichnet, daß einzelne Auflagen (32) aus absorbierendem Stoff mit Abstand voneinander auf den mit Klebstoff beschichteten Streifen (16) aufgebracht werden.

8. Verfahren nach Anspruch 6 oder 7, dadurch gekennzeichnet, daß der mit Klebstoff beschichtete Streifen die Aushärtzone (40) vor dem Aufbringen der Auflage(n) (32) durchläuft.

9. Verfahren nach Anspruch 1, dadurch gekennzeichnet, daß der Klebstoff ein heißschmelzender Klebstoff ist, der bei einer erhöhten Temperatur auf den Verstärkungsstreifen (16) aufgetragen wird, wobei das Abkühlen des Klebstoffes in der Konditionierzone (40) erfolgt und das Aufbringen der Auflage (32) auf den mit Klebstoffbeschichteten Streifen erfolgt, nachdem der Klebstoff abgekühlt worden ist.

10. Verfahren nach Anspruch 9, dadurch gekennzeichnet, daß die Auflage (32) aus einem aushärtbaren Schaummaterial besteht, wobei das Verfahren das Leiten des mit einer oder mehreren Auflagen versehenen Streifens durch eine Aushärtstufe (40) vor dem Eintritt in die Schutzpapier-Aufbringzone (50) umfaßt.

11. Verfahren nach einem der Ansprüche 1 bis 10, dadurch gekennzeichnet, daß es das Leiten des endlosen Klebeverbandes von der Schutzpapier-Aufbringzone (50) zu einer Schneidzone (60) und das Schneiden zu Einzelverbänden einer gewünschten Form und Größe in der Schneidzone umfaßt.

12. Verfahren nach Anspruch 11, dadurch gekennzeichnet, daß das Schneiden durch eine Schneidstempelanordnung (62) erfolgt.

## Revendications

1. Procédé de fabrication d'un pansement adhésif comportant des étapes consistant à
- faire passer en continu une bande support (16) à travers une zone (20) d'application d'un adhésif ;
- appliquer un adhésif sur l'une des faces de la bande support (16) lorsqu'elle passe en continu à travers cette zone (20) pour former une bande revêtue d'adhésif ;
caractérisé par le fait qu'il comporte les étapes consistant à
- faire passer en continu la bande, revêtue d'adhésif, depuis la zone (20) d'application de l'adhésif, à travers une zone (40) de conditionnement de l'adhésif pour conditionner l'adhésif ;
- avant ou après la zone (40) de conditionnement de l'adhésif, faire passer en continu la bande, revêtue d'adhésif, à travers une zone (30) d'application de rembourrage où au moins un rembourrage (32) est appliqué sur la face de la bande support revêtue d'adhésif de façon telle que des portions de l'adhésif ne sont pas recouvertes par le rembourrage, formant ainsi une bande rembourrée ;
- amener en continu et directement la bande rembourrée dans une zone (50) d'application d'un papier à enlever où le papier à enlever (52, 54) est appliqué sur au moins l'adhésif, pour former un pansement adhésif continu.

2. Procédé selon la revendication 1, caractérisé par le fait que l'adhésif est un adhésif qui durcit sous l'action de la pression, le durcissement de l'adhésif s'opérant alors dans la zone (40) de conditionnement de l'adhésif.

3. Procédé selon la revendication 2, caractérisé par le fait que le rembourrage (32) est en matériau mousse durcissable, la bande revêtue d'adhésif passant à travers la zone (30) d'application du rembourrage avant de passer à travers la zone de conditionnement (40) de sorte que le durcissement du matériau mousse s'effectue également dans la zone de conditionnement (40).

4. Procédé selon la revendication 3, caractérisé par le fait que le matériau mousse est appliqué en continu sur la bande support (16).

5. Procédé selon la revendication 3, caractérisé par le fait que le matériau mousse est appliqué de façon intermittente sur la bande support (16).

6. Procédé selon la revendication 2, caractérisé par le fait que le rembourrage (32) est en tissu absorbant et qu'il est appliqué sous forme d'une bande continue sur la bande (16) revêtue d'adhésif.

7. Procédé selon la revendication 2, caractérisé par le fait que des rembourrages individuels (32) en tissu absorbant sont appliqués de façon intermittente sur la bande (16) revêtue d'adhésif.

8. Procédé selon la revendication 6 ou la revendication 7, caractérisé par le fait que la bande revêtue d' adhésif passe à travers la zone de durcissement (40) avant application du/des rembourrage(s) (32) sur cette bande.

9. Procédé selon la revendication 1, caractérisé par le fait que l'adhésif est un adhésif fondant à chaud appliqué sur la bande support (16) à température élevée, le refroidissement de l'adhésif s'effectuant dans la zone de conditionnement (40) et l'application du rembourrage (32) sur la bande revêtue d'adhésif se faisant après refroidissement de l'adhésif.

10. Procédé selon la revendication 9, caractérisé par le fait que le rembourrage (32) est en matériau mousse durcissable, le procédé incluant l'étape consistant à faire passer la bande rembourrée à travers un étage de durcissement (40) avant de la faire entrer dans la zone (50) d'application du papier à enlever.

11. Procédé selon l'une de quelconques revendications 1 à 10 inclusivement, caractérisé par le fait qu'il comporte l'étape consistant à faire passer le pansement adhésif continu, de la zone (50) d'application d'un papier à enlever, à une zone de découpe (60) et à le découper dans la zone de découpe en pansements individuels d'une forme et d'une dimension désirées.

12. Procédé selon la revendication 11, caractérisé par le fait que la découpe s'effectue au moyen d'un dispositif de découpe à matrice (62).
